(19) Europäisches Patentamt
European Patent Office
Office européen des brevets

(11) EP 2 748 143 B1

(12) **EUROPEAN PATENT SPECIFICATION**

(45) Date of publication and mention of the grant of the patent:
**16.08.2017 Bulletin 2017/33**

(21) Application number: **12759030.5**

(22) Date of filing: **22.08.2012**

(51) Int Cl.:
***C07C 323/14*** *(2006.01)* ***C07C 323/19*** *(2006.01)*
***C07F 15/00*** *(2006.01)*

(86) International application number:
**PCT/EP2012/066357**

(87) International publication number:
**WO 2013/026885 (28.02.2013 Gazette 2013/09)**

(54) **MONOMERIC AND OLIGOMERIC UNSATURATED VICINAL DITHIOETHERS AND THEIR USE FOR SELECTIVE PALLADIUM (II) SEPARATION FROM SECONDARY RAW MATERIALS**

MONOMERE UND OLIGOMERE UNGESÄTTIGTE VICINALE DITHIOETHER UND IHRE VERWENDUNG ZUR SELEKTIVEN PALLADIUM (II)-TRENNUNG VON SEKUNDÄRROHSTOFFEN

DITHIOÉTHERS VICINAUX MONOMÉRIQUES ET OLIGOMÉRIQUES INSATURÉS ET LEUR UTILISATION POUR LA SÉPARATION SÉLECTIVE DE PALLADIUM (II) À PARTIR DE MATIÈRES PREMIÈRES SECONDAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.08.2011 EP 11178303**

(43) Date of publication of application:
**02.07.2014 Bulletin 2014/27**

(73) Proprietor: **Universität Potsdam**
**14469 Potsdam (DE)**

(72) Inventors:
- **HOLDT, Hans-Jürgen**
  **19412 Jülchendorf (DE)**
- **TRAEGER, Juliane**
  **14467 Potsdam (DE)**

(74) Representative: **Müller & Schubert Patentanwälte**
**Schlüterstrasse 37**
**10629 Berlin (DE)**

(56) References cited:

- **V. P. ANANIKOV ET AL: "Sulfur-containing alkenes-A new class of chelating ligands: Synthesis, coordination to palladium, and structure of the resulting complexes", RUSSIAN JOURNAL OF ORGANIC CHEMISTRY, vol. 45, no. 12, 1 December 2009 (2009-12-01), pages 1743-1754, XP55042035, ISSN: 1070-4280, DOI: 10.1134/S107042800912001X cited in the application**
- **BERNARDIS F L ET AL: "A review of methods of separation of the platinum-group metals through their chloro-complexes", REACTIVE & FUNCTIONAL POLYMERS, ELSEVIER SCIENCE PUBLISHERS BV, NL, vol. 65, no. 3, 1 December 2005 (2005-12-01), pages 205-217, XP027640396, ISSN: 1381-5148 [retrieved on 2005-12-01] cited in the application**
- **H.J. Holdt: "Thiacrown compounds with 1,2-dicyano-l, 2-dithioethene units", Pure & Appl. Chem, 1 January 1993 (1993-01-01), pages 477-482, XP55042323, Retrieved from the Internet: URL:http://pac.iupac.org/publications/pac/pdf/1993/pdf/6503x0477.pdf [retrieved on 2012-10-26] cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- HARTLEY F R ET AL: "SYSTEMATICS OF PALLADIUM(II) AND PLATINUM(II) DITHIOETHER COMPLEXES. THE EFFECT OF LIGAND STRUCTURE UPON THE STRUCTURE AND SPECTRA OF THE COMPLEXES AND UPON INVERSION AT COORDINATED SULPHUR", INORGANICA CHIMICA ACTA, ELSEVIER BV, NL, vol. 35, no. 2, 1 January 1979 (1979-01-01), pages 265-277, XP008000955, ISSN: 0020-1693, DOI: 10.1016/S0020-1693(00)93450-9 cited in the application
- S. HOLGER EICHHORN ET AL: "Metal ion mediated mesomorphism and thin film behaviour of amphitropic tetraazaporphyrin complexes", JOURNAL OF MATERIALS CHEMISTRY, vol. 11, no. 6, 1 January 2001 (2001-01-01), pages 1576-1584, XP55042070, ISSN: 0959-9428, DOI: 10.1039/b100112o
- SHOLTO ALAN ET AL: "Spectroscopy, binding to liposomes and production of singlet oxygen by porphyrazines with modularly variable water solubility", PHOTOCHEMISTRY AND PHOTOBIOLOGY, WILEY-BLACKWELL PUBLISHING, INC, US, vol. 84, no. 3, 1 May 2008 (2008-05-01), pages 764-773, XP002589001, ISSN: 0031-8655, DOI: 10.1111/J.1751-1097.2007.00268.X [retrieved on 2008-01-15]
- NAMBARA: "Studies on Chemotherapeutics for Acid-fast Bacilli. XIII. On some Compounds Related to vinyl Sulfones and their Bacteriostatic and Chemical Activities", YAKUGAKU ZASSHI, vol. 75, 1955, pages 1560-1564, XP009164061,
- MURSAKULOV, I. G.; RAMAZANOV, E. A.; KERIMOV, F. F.; ABBASOV, I. M.; ZEFIROV, N. S: "Bromination of 2,3-unsaturated 1,4-disulfides", JOURNAL OF ORGANIC CHEMISTRY USSR (ENGLISH TRANSLATION), vol. 26, 1990, pages 113-116, XP009164059,
- KALKAN, AYFER; BAYIR, ZEHRE A.: "Synthesis and Characterisation of Unsymmetrical Porphyrazines Containing Bis(hydroxyethylthio) Substituents", MONATSHEFTE FÜR CHEMIE, vol. 134, 2003, pages 1555-1560, XP002686010,
- THOMAS SCHWARZE ET AL: "Systematic Investigation of Photoinduced Electron Transfer Controlled by Internal Charge Transfer and Its Consequences for Selective PdCl 2 Coordination", CHEMISTRY - A EUROPEAN JOURNAL, vol. 16, no. 6, 8 February 2010 (2010-02-08), pages 1819-1825, XP55042300, ISSN: 0947-6539, DOI: 10.1002/chem.200902281
- BOROWITZ, IRVING J.; READIO, JOSEPHINE D.; LI, VEN-SHUN: "The preparation and properties of neutral diamide ionophores for group IIA metal cations-II", TETRAHEDRON, vol. 40, no. 6, 1984, pages 1009-1016, XP0028086945,
- Ralf Heber: "Metallchelate ungesättigter 1,2-Dithioäther", Journal f. prakt. Chemie, 1 January 1976 (1976-01-01), pages 19-25, XP55042346, Retrieved from the Internet: URL:http://onlinelibrary.wiley.com/store/10.1002/prac.19763180104/asset/19763180104_ftp.pdf?v=1&t=h8r213gv&s=c6eac15d7817cd5bd e0b7640886680a044da23a9 [retrieved on 2012-10-26]
- Juliane Traeger ET AL: "Complexation of Palladium(II) with Unsaturated Dithioethers - A Systematic Development of Highly Selective Ligands for Solvent Extraction", EUROPEAN JOURNAL OF INORGANIC CHEMISTRY., vol. 2012, no. 14, 28 February 2012 (2012-02-28), pages 2341-2352, XP055271231, DE ISSN: 1434-1948, DOI: 10.1002/ejic.201101406

## Description

The present invention relates to monomeric unsaturated vicinal dithioethers. Furthermore the present invention relates to the use of unsaturated vicinal dithioethers according to the invention in methods for selective palladium(II) separation from secondary raw materials.

In the following for the novel unsaturated vicinal dithioethers the terms compound and ligand are used synonymousyl.

The recovery of platinum group metals (PGM) from secondary sources has gained increasing importance, especially in countries without platinum-group metal mines. As measured by the annual demand palladium is one of the most important platinum-group metal. In industrial process streams it occurs in the divalent oxidation state and forms chloro complexes, with the tetrachloropalladate anion, $[PdCl_4]^{2-}$, being the most common species.

Nowadays one of the secondary sources for selective recovery of palladium is derived from the production and recovery of automotive catalysts. But this is only one application area for the compounds and methods of the invention.

The selective recovery of Pd(II) from automobile catalysts is a very challenging task because the sources usually are acidic and oxidizing media.

The most common automotive catalysts consist of ceramic monoliths, that are mainly coated with aluminium oxide but also rare earth and base metal oxides as well as alkaline earths. Furthermore platinum group metals are fixed at the surface of that washcoat. Beside palladium, these are particularly platinum and rhodium.

In the state of the art it is known to leach the automobile catalysts with a strongly oxidising acidic medium containing chloride, like aqua regia, in order to obtain the PGM chloro complexes.

The liquid gained in this hydrometallurgical way, naturally also contains a wide spectrum of other metals, some of which are in a large excess. Moreover, compared to feed solutions originating from pyrometallurgical enrichment processes, the concentration of the PGM is relatively low (some hundred milligrammes per litre).

Nowadays the separation of the individual platinum group metals like palladium is mainly done via extraction processes namely solvent extraction and solid phase extraction methods.

In solvent extraction methods the palladium ions contained in an aqueous liquid phase are chelated with an appropriate ligand, transferred to an organic phase and afterwards released from the complex and thereby separated.

However solid phase extraction methods offer a number of advantages over solvent extraction methods, caused by a less problematic handling of the solid material compared to flammable and volatile solvents. Moreover, in solid phase extraction methods the formation of stable emulsions is avoided, which makes it a lot easier to separate the two phases and reduces the loss of extractant.

An alternative approach consolidates selective extractants used in solvent extractions with the benefits of solid phase operations. In solvent impregnated resins (SIRs) the ligand is physically impregnated into a polymeric support, making preparation of these materials rather convenient.

In these methods the ligand used mainly accounts for the selectivity, whereas the support influences the extraction kinetics and should provide certain properties, such as a high surface area and porosity as well as chemical and mechanical resistance [1]. Commonly one reverts to the same solid supports as they are used in ion exchange resins. In this way, solid phase extraction studies on SIRs can be also seen as a preliminary step to testing with covalently bound extractants.

Considering the composition of a solution from a recovery process, the development of a selective extractant for Pd(II) is a challenging task. The extractant has on the one hand to be selective in respect of the metal and on the other hand has to withstand the reaction conditions that can be strong acidic.

Therefore it is an aim of the invention to provide novel compounds that can be utilized in extraction processes for palladium(II) and to investigate their usefulness in different extraction methods.

Malik and Paiva [2] have given an overview of relevant works dealing with the separation of Pd(II), Pt(IV) and Rh(III) from chloride solutions. A major drawback of most extracting agents presented there for Pd(II) is the insufficient selectivity.

It was reported that especially when using ion exchangers the separation from Pt(IV) and Rh(III) is not taking place, but also with solvent extractants like malonamide or organophoshine ligands Pd(II) is not separated during the extraction step.

In industrial processes in the state of the art it is known to recover palladium via solvent extraction with hydroxyoximes or long chain thioethers through the formation of inner-sphere complexes [3,4,5].

One disadvantage of the known methods is that due to slow extraction rates the equilibration times of such known systems are quite long [4,5]. These equilibration times can be reduced with the help of phase transfer catalysts, like amines in the case of β-hydroxyoximes [5], but that decreases the selectivity towards other metals. In addition when using long chain thioethers, stripping the palladium from the organic phase is found to be difficult [5].

General requirements of a solvent extractant suitable for use in commercial operations have been specified 1987 by Tavlarides et al. [6]. Besides the key focus - a selective extraction of the metal with a high loading capacity

under the required pH - they include aspects like acceptable equilibrium times, an easy stripping of the loaded metal from the organic phase, the stability of the extractant throughout the several stages and a high solubility of the extractant in the organic phase (mostly aliphatic and aromatic diluents) along with a low solubility in the aqueous phase. Moreover the extractant should be non-flammable, non-volatile and non-toxic for security and environmental reasons as well as relatively inexpensive.

In the state of the art thioethers are widely known to be soft donors and to form complexes with a range of transition metals. Dithioethers based on (cis)-1,2-dithioethene possess a rigid chelating unit, which perfectly meets the geometrical demand for the square planar complexation of Pd(II) [7]. Recently Ananikov et al. have reported on sulfur-containing alkenes and their Pd(II) complexes [8].

To overcome the drawbacks of the state of the art one approach is to design ligands with a reduced electron density on the sulfur atoms. This can be achieved by introducing electron withdrawing groups on the double bond, such as cyano groups, or the double bond being part of an aromatic ring.

Studies on maleonitrile-dithiocrown ethers have shown that they form stable Pd(II) complexes and have also demonstrated their potential to extract Pd(II) with a good selectivity towards other soft metal ions [9,10]. Another important attribute is their high surface activity caused by the ether groups. Yet because of the low yields in their synthesis these compounds have never been used on a large scale for solvent extraction.

As Pd(II) is coordinated exocyclically via the two sulfur atoms, the steric design does not stringently need to contain macrocycles. Hence the open-chain equivalents, which are easier to synthesise, are promising ligands to be utilised in industrial processes.

Pd(II) complexes of 1,2-bis(methylthio)maleonitrile [11], 1,2 bis(methylthio)-benzene [7,11,12] and 1,2-bis(methylthio)-4-methylbenzene [12] have already been investigated earlier, but the ligands themselves have not received any attention concerning a possible applicability in solvent extraction.

Since the complexation of the metal occurs at the interface or the aqueous phase, it is important that the extractant used is not too nonpolar - otherwise it takes very long until the equilibrium establishes.

In the solid phase extraction methods the operation can be done in a column-process realising a high contact surface without additional mixing [13]. Usually solid phase extraction is associated with ion exchangers, but often these materials show a much poorer selectivity than coordinating extractants.

Negatively charged chloro complexes of Pd(II) and Pt(IV) and to a lesser extent also Rh(III) are often co-extracted when using anion exchangers [14, 15]. With so-called selective chelating ion exchange resins - polymers with covalently bound side chains containing chelating functional groups with sulfur, nitrogen or oxygen donor atoms - different extraction mechanisms can occur. Depending on the acidity of the feed solution an inner-sphere coordination mechanism or outer sphere ionic mechanism predominates [16, 17].

Yet the linking of the functional groups to the backbone of the resin is complex and time-consuming, resulting in a costly production process. For this reason there is only a small number of those resins available on the market [13, 1]. In addition, these materials are often too hydrophobic and sometimes the selectivity of the coordinating ligand is not preserved [18].

Until now, no compounds have been disclosed that are suitable as complex forming agents for the extraction of Pd(II) at different pH values from a wide range of secondary raw materials.

It is therefore an aim of the present invention to provide such complex forming compounds that can be utilized in solid phase extraction processes of Pd(II) and be used as solid phase extractants in solvent impregnated resin extraction processes.

## Short description of the invention

Therefore it is an object of the invention to provide compounds that are comparably easy and cost effective to synthesise and at the same time are ligands to be utilised in industrial processes.

First and foremost to be utilized in industrial applications a compound is preferred that can be synthesized in a method with few steps and most preferred in a one-pot synthesis.

To solve the problem of the invention the inventors have provided a number of novel monomeric vicinal unsaturated dithioethers and have investigated the use thereof in solvent extraction and solid phase extraction of Pd(II). To show the usefulness of the novel unsaturated vicinal dithioethers their potential for selective extraction of Pd(II) from different media was investigated. In a part of these experiments the acidic and oxidizing media from the recovery of automobile catalysts were simulated. The present disclosure describes unsaturated vicinal dithioethers of general formula I:

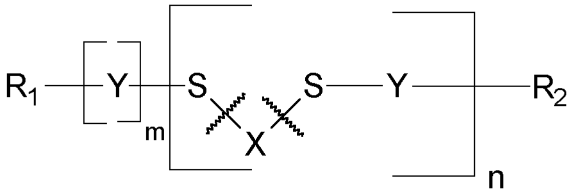

I

wherein n is an integer from 1 to 12, wherein m is 0 or 1, wherein X is selected from

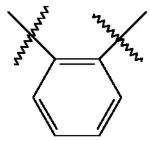

,

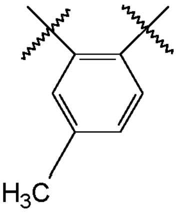

or

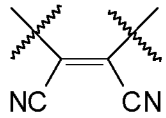

wherein Y is selected from the group consisting of

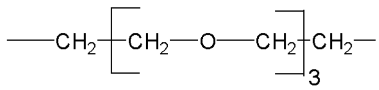

,

$-CH_2-CH_2-CH_2-$, $-CH_2-CH_2-O-$, $-O-CH_2-CH_2-$, $-CH_2-CH_2-O-CH_2-$, and $-CH_2-O-CH_2-CH_2-$.
wherein, under the proviso that n is 1, m is 1 and $R_1$ and $R_2$ each are H; wherein, under the proviso that n > 1, $R_1$ and $R_2$ are selected from the group consisting of H, Na, Cl, and Br,or $R_1$ and $R_2$ together form a bond making up a cycle.

The problem of the invention is solved by providing unsaturated vicinal dithioethers of general formulae (2) and (4):

1,2-bis(2-methoxyethylthio)-4-methylbenzene (2)

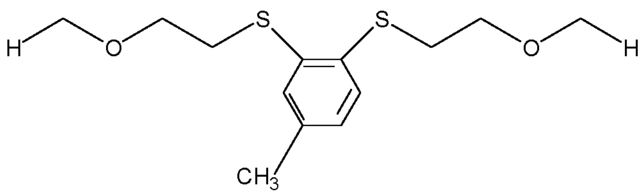

,

and

1,2-bis(2-methoxyethylthio)benzene (4)

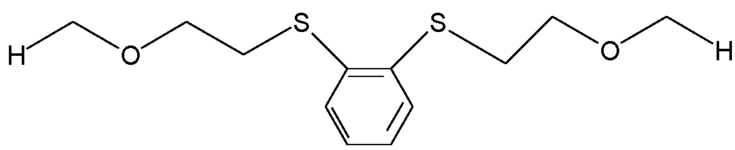

.

In an particularly preferred embodiment of the invention a compound according to the invention is used in the

separation of Pd(II) from secondary raw materials.

Especially preferred is the use of a compound according to the invention, wherein the separation is a solvent extraction method.

In a preferred embodiment a compound according to the invention is used in a solid phase extraction method.

Preferred is an embodiment of the invention wherein the use of a compound according to the invention is a solvent impregnated resin (SIR) extraction method.

Particularly preferred is the use according to the invention wherein the secondary raw material is derived from the production or recovery of automobile catalysts.

The problem of the invention is furthermore solved by providing a solid support comprising a compound according to the invention.

In a preferred embodiment of the invention the solid support is silica gel or a polymeric resin.

Particularly preferred is a support, wherein the polymeric resin is a macroporous styrene-divinylbenzene copolymer.

In contrast to thioethers of the state of the art the novel compounds are not volatile and do not have the characteristic odour, which is advantageous for practical application.

To investigate the usefulness of the novel unsaturated 1,2-thioethers of the invention solvent extraction batch experiments with the unsaturated dithioethers (1) to (6) and various reference compounds were performed. Compounds (1), (3) and (5)-(6) are state of the art.

All tests were carried out in a chloroform / water system, where the extractant was present in excess in the organic phase. The composition of the aqueous phase, which contained Pd(II), has been progressively modified from a model solution closer to industrial conditions.

The invention is further detailed by reference to the accompanying drawings.

## Brief discussion of the drawings

Figure 1 shows the solvent extraction kinetics of the dithioethers (2), (4), (5), and (6) compared to dihexylsulfide and Aloxime®840 in $CHCl_3/H_2O$ = 1/1 ($c(L)_o = 10^{-2}$ M, $c_0(Pd)_w = 10^{-4}$ M, $c(Cl)_w = 1.5 \times 10^{-2}$ M). The extraction yield E was determined by measuring $c(Pd)_w$ with ICP OES.

Figure 2 shows the solvent extraction kinetics of dithioether (2), 4-methylbenzodithio-12-crown-4 and 1,2-bis(methylthio)-4-methylbenzene in $CHCl_3/H_2O$ = 1/1 ($c(L)_o = 10^{-2}$ M, $c_0(Pd)_w = 10^{-4}$ M, $c(Cl)_w = 1.5 \times 10^{-2}$ M). The extraction yield E was determined by measuring $c(Pd)_w$ with ICP OES.

Figure 3 shows the dependency of the extraction yield *E* of maleonitrile-dithio-12-crown-4, dithioethers (2), (4) and (6) on the HCl concentration added to the aqueous phase ($CHCl_3/H_2O$ = 1/1, $c(L)_o = 10^{-2}$ M, $c_0(Pd)_w = 10^{-4}$ M, $c_0(Cl)_w = 1.5 \times 10^{-2}$ M). The extraction yield *E* is determined by measuring $c(Pd)_w$ with ICP OES.

Figure 4 shows the results of a solvent extraction batch experiment with a solution gained by leaching of an automotive catalyst with aqua regia (subsequent dilution with the same volume of water) and extractant (4) in chloroform. Composition of the feed solution (dark grey) and organic phase (light grey) as determined with ICP OES ($c(\mathbf{4})_o = 10^{-2}$ M, $H_2O/CHCl_3$ = 1/1).

Figure 5 shows SPE kinetics of the SIRs in batch tests ($10^{-4}$ M Pd(II), 0.02 M HCl).

Figure 6 shows the elution of the loaded SIRs (see Table 2 for the initial Pd content) in batch tests with 0.5 M thiourea, 0.1 M HCl.

## Detailed description of the invention

Figure 1 clearly demonstrates the ability of the ligands (2), (4) and (6) to transfer Pd(II) from the aqueous into the organic phase. Compared to the two industrially used extractants dihexylsulfide [19] and Aloxime®840 [20] all of compounds of the invention containing 2-methoxyethyl end groups establish the extraction equilibrium extremely fast. Especially (2) and (4), which show extraction yields near 100 % within only 30 minutes, are preferred compounds according to the invention.

Compound (5) formed an insoluble precipitate, which accumulated in between the phases and could not be analysed.

Thus, although the palladium content of the aqueous phase instantly drops when getting in contact with (5), it is unsuitable to be used as an extractant. Surprisingly the compounds (1) and (3) did not extract any Pd(II). This was caused by their high water solubility, as revealed by scanning of both phases via UV/Vis spectroscopy. Both ligands form palladium complexes, but under the conditions chosen their concentration in the aqueous phase has been high enough to keep the palladium there.

Figure 2 illustrates the influence of the end groups on the extraction kinetics within a series of 1,2-dithio-4-methylbenzene derivatives: the acyclic 1,2-bis(2-methoxyethylthio)-4-methylbenzene (2) has been tested in comparison to the analogue crown ether 4-methylbenzodithio-12-crown-4 [21] and the non-polar acyclic 1,2-bis-(methylthio)-4-methylbenzene [12]. All of them exhibit very good extraction yields, but the time taken to reach the extraction equilibrium differs widely. Using the non-polar 1,2-bis(methylthio)-4-methylbenzene the extraction takes the longest.

The reaction rate increases according to an enhancement of the surface activity in the order of: methyl < 2 methoxyethyl group. 4-Methylbenzodithio-12-crown-4, possessing a similar polarity to 2, reacts fastest. Without intending to be bound to a theory this difference in kinetics could be explained by the rigidity of the crown ether. Compared to the open-chain ligand (2), it features a better preorganisation for a complexation of Pd(II). Interestingly the extraction yield of the dithiomaleonitrile derivative (6) is less than that of the extractants (2) and (4) based on the dithiobenzene unit.

For media containing a high concentration of hydrochloric acid, like the streams in platinium group metals refining industries, the extractants used have to be strong enough to compete with $Cl^-$ for a complexation at Pd(II).

As can be seen from Figure 3 there is a huge difference in the extraction performance between extractant (2), (4) and (6). The extraction yield of the dithiomaleonitrile derivative dramatically decreases even by the addition of very small quantities of HCl. At a concentration level of 0.26 M HCl ligand (6) extracts almost no Pd(II) at all. The same trend is observed when using the corresponding crown ether maleonitrile-dithio-12-crown-4 [22], although it is shifted to higher HCl concentrations. Being more preorganised the crown ether is a stronger chelating agent than the open-chain analogue (6). However, utilising HCl concentrations higher than 1 M also depresses the extraction yield to negligible values.
In contrast, extractants (2) and (4) constantly exhibit extraction yields near 100 % even at extremely high HCl concentrations.

Figure 4 demonstrates the capability of extractant (4) under conditions close those in recovery proceses for automotive catalysts.

A solution was extracted, which was gained by leaching an automotive catalyst with aqua regia and diluting that 1:1 with water. Beside a HCl concentration of nearly 4.5 M, this solution did not only contain a higher concentration of Pd(II) but also Pt(IV) and Rh(III), as well as a number of base metals, partially in 10 fold excess.
The selectivity of compound (4) was found to be excellent. The organic phase contained 133 mg/L palladium, whereas the concentration of all the other metals was smaller than 0.2 mg/L. This experiment also demonstrates the redox stability of the system under strongly oxidising conditions.

This shows that the novel chelating compounds (2) and (4) can be utilised for the selective coordination of Pd(II).

They proved to have a high stability towards oxidation, which makes them interesting for application in solvent extraction industries.

In batch experiments it were the ligands (2) and (4), which particularly substantiated the capability to be used as solvent extractants. They are based on 1,2-dithio-4-methylbenzene and o-dithiobenzene, respectively and contain 2-methoxyethyl side-chains.

Compared to conventionally used extractants like dihexylsulfide or hydroximes the novel ligands enable a drastic increase of the reaction rate. The influence of the varying backbones of our ligands on the complex stabilities reflects in a dramatic disparity of the extraction performance in the presence of chloride.

Like mentioned above, for an application in industry it is important that the extractant is cheap to produce, very stable against oxidation and also able to extract Pd(II) out of a medium containing a high content of hydrochloric acid. On the other hand the complex formed should not be too stable to enable a fast elution. These criteria are perfectly met by ligand (4) rendering it the extractant of choice.

With the above referenced results the high potential of compound (4) by selectively extracting Pd(II) in solvent extraction methods out of a solution of a leached automotive catalyst was demonstrated, which also contained a high concentration of other PGM and base metals as well as hydrochloric acid.

In the following the usefulness of a selection of the compounds according to the invention was investigated in the solid phase extraction of Pd(II). In these experiments three different kinds of solid supports were impregnated with the ligands of the invention.

To this end several extractants, including compound 1,2-bis(2-methoxyethylthio)benzene (4) has been applied onto silica gel and amberlite XAD-2.

Table 1 shows the composition of the SIRs that were prepared utilizing the compounds according to the present invention.

**Table 1** Denotation and composition of SIRs.

| Denotation | Extractant | Solid Support | $\frac{m_{Extractant}}{m_{Support}}$ | $x_S \left(\frac{mmol}{g}\right)$ |
|---|---|---|---|---|
| XAD2-4a | 4 | XAD 2 | 0.79 | 3.4 |
| XAD2-4b | 4 | XAD 2 | 1.18 | 3.9 |

wherein:

***mExtractant***

***mSupport*** is the ratio between mass of extractant and mass of solid support and xs is the sulphur content. The measured sulfur content xs directly depends on the content of dithioether units

The inorganic, hydrophilic silica gel is a typical supporting material for solid support extraction materials [23, 24].

Amberlite XAD-2, a macroporous styrene-divinylbenzene copolymer, is a commonly used, organophilic substrate that adsorbs the ligands via n-n-dispersion forces.

Generally XAD resins are more stable against acid and alkali solutions than $C_{18}$-bonded silica gel, which also had been widely used as adsorbent.

The batch experiments performed with the SIRs of Table 1 revealed that the extraction behaviour of the SIRs varies widely (Fig. 5). The extractant on the surface of the solid support determines the extraction yields.

In the context of the investigations of the momomeric unsaturated dithioethers of the present invention it was shown that due to the higher electron withdrawing effect of the dithiomaleonitrile backbone the stability of the respective Pd(II) complex formed is less than that of a dithiobenzene derivative. On the other hand the percentage of metal extracted during one extraction step also depends on the hydrophilicity of the extractant, which is varied via the number of oxygen atoms within the chains connecting the chelating dithioether units. The SIR XAD2-4a comprising the monomeric extractant, exhibited an extraction yield of 69.8%. XAD2-4b was overloaded and the extractant washed off the solid support, which resulted in an oily film at the surface of the aqueous phase. Hence this SIR was not used for further experiments. That shows that amount of the extractant chosen for XAD2-4a is already near the maximal possible value.

Furthermore in successive batch extraction experiments the effective Pd(II) capacity $q_{Pd}$ of the SIRs was determined according to equation (2), where n is the number of extraction steps until the SIR was fully loaded, m is the mass of the SIR and $V_i$ the volume of the aqueous phase.

$$q_{\mathbf{Pd}} = \frac{\sum_{i=1}^{n}\left([\mathbf{Pd}]_0 - [\mathbf{Pd}]_i^{t}\right) \cdot V_i}{m} \qquad (2)$$

In Table 2 the results of these experiments are shown. Table 2 shows the contact times for each extraction step, the number of steps and the palladium capacities as determined via batch experiments ($10^{-4}$ M Pd(II), 0.02 M HCl).

**Table 2**

| **SIR** | **$t_{contact}$ (min)** | **n** | **$q_{Pd}$ (mmol/g)** |
|---|---|---|---|
| XAD2-4a | 360 | 43 | 0.44 |
| XAD2-4b | - | - | - |

The effective capacities are generally lower than theoretically possible, relating to the content of sulfur $x_s$ listed in Table 1 (under the assumption that two sulfur atoms are needed to bind one Pd(II)). The highest loading capacity has been achieved by XAD2-4a, which also contains the highest sulfur content. It is comparable to the loading capacity reported for SuperLig® from IBC (0.52 mmol/g) [25].

To show the usability of the compounds of the present invention in solid phase extraction materials it is further shown that the solid supports impregnated with the compounds of the invention can be used for many extraction cycles.

For regeneration the extractant is treated with an eluent, which re-extracts the metal into an aqueous phase. In solvent extraction experiments with the monomeric extractant (4), a mixture of thiourea in hydrochloric acid was found to be the most effective stripping agent [30]. Hence this solution was also used for the elution of Pd(II) from the SIRs

via successive batch experiments with 3 mL eluent at a time.

As seen from Fig. 6, showing the Pd(II) content of each dose, the thiourea solution is a highly effective re-extractant that recovers the major part of the metal already in the first elution step. In the cases of XAD2-4a the total percentage of Pd(II) that could be recovered was only 68% for both SIRs. It is possible that the remaining Pd(II) had migrated into regions that have been inaccessible for the eluent.

These experiments showed that the compounds of the present invention can be utilized as selective extractants for Pd(II) in solvent impregnated resins and have a potential to be used as solid phase extractants.

The problem of the present invention is solved by providing the monomeric unsaturated vicinal dithioethers of the present invention the synthesis and the investigation thereof is described in the following. Additionally the preparation of the SIRs provided in the present invention is disclosed.

## Syntheses of the novel unsaturated vicinal dithioethers

### Synthesis of the monomeric compounds (1)-(6)

General: All reactions were carried out in dry solvents under an argon atmosphere. The melting points (m.p.) were measured with a capillary. NMR spectra of the compounds (1)-(6) were recorded with a Bruker Avance-300 spectrometer. The $^1$H chemical shifts are reported relative to the TMS signal (0 ppm) and the $^{13}$C chemical shifts relative to the solvent signal $CDCl_3$ (77 ppm). The assignments of the NMR signals were carried out under using 2D-NMR experiments HMBC and HMQC. IR spectra were recorded on a Thermo Nicolet NEXUS FTIR instrument. UV/Vis spectroscopic measurements were done on a Perkin Elmer Lambda 950 spectrophotometer using quartz cuvettes. The EI MS spectra were recorded using Thermo Quest SSQ 710. Elemental analyses (C,H,N,S) of the ligands 1-6 were performed with an Elementar Vario EL elemental analyser. The Pd content of the complexes $[PdCl_2(L)]$ was determined by diluting 1 mg in 1 mL 65 % $HNO_3$ and measuring on an Optima 5300 DV ICP OES from Perkin Elmer ($\lambda$ = 340.458 nm).

### General procedure for the synthesis of compounds (1)-(4):

Benzene-1,2-dithiol or toluene-3,4-dithiol respectively (9.5 mmol) in ethanol (3 mL) was added to a solution of sodium (20 mmol) in ethanol (21 mL). The resulting solution was heated under reflux and 20 mmol of 2-bromoethanol or 2-chloroethylmethylether respectively were added. The solution was stirred under reflux for 15 hours. After that the precipitate was removed and the solvent was evaporated. The yellow, oily residue was purified by column chromatography on silica gel using chloroform as an eluent to afford (1), (2) and (4) as colourless oils and (3) as white crystals.

The maleonitril-dithioethers (5) and (6) were prepared by alkylation of disodium (*Z*)-1,2-dicyanoethene-1,2-dithiolate [26] with 2-bromoethanol and 2-chloroethylmethylether, respectively in the presence of NaI following a procedure reported by Lange and coworkers [27].

### 1,2-bis(2-hydroxyethylthio)-4-methylbenzene (1) (State of the art)

Yield: 2.16 g (93 %); Rf=0.12 ($CHCl_3$); $n_D^{20}$: 1.621; $^1$H NMR (300 MHz, $CDCl_3$, 25 °C, TMS): $\delta$=7.32 (d,$^3J$(H,H)=8 Hz, 1H; CHC*H*CS), 7.21 (s, 1H; CC*H*CS), 7.01 (d, $^3J$(H,H)=8.0 Hz, 1H; CC*H*CH), 3.73 (t, $^3J$(H,H)=5.7 Hz, 2H; $CH_2C^hH_2O$), 3.67 (t, $^3J$(H,H)=5.7 Hz, 2H; $CH_2C^jH_2O$), 3.12 (t, $^3J$(H,H)=5.7 Hz, 2H; $SC^gH_2CH_2$),), 3.07 (t, $^3J$(H,H)=5.7 Hz, 2H; $SC^iH_2CH_2$), 2.65 (brs, 2H; O*H*), 2.32 ppm (s, 3H; $CH_3C$); $^{13}$C NMR (75 MHz, $CDCl_3$, 25 °C, TMS): $\delta$=138.1 ($CH_3CCH$), 137.5 ($CHC^aS$), 132.5 ($CHC^fS$), 132.1 (CH*C*HCS), 131.1 (C*C*HCS), 128.4 (C*C*HCH), 60.0 ($CH_2C^hH_2O$), 59.8 ($CH_2C^jH_2O$), 38.5 ($SC^gH_2CH_2$), 37.7 ($SC^iH_2CH_2$), 21.0 ppm ($CH_3C$); IR (KBr): $\tilde{\nu}$=3383 (O-H), 2921 (C-H), 2873 (C-H), 1459 (C-C), 1063 (C-OH), 1039 (C-OH), 1012 $cm^{-1}$ (C-OH); UV/Vis ($H_2O$): $\lambda_{(max)}$ ($\varepsilon$)=296 (1720), 247 (11704), 216 nm (22061 $mol^{-1}dm^3cm^{-1}$); MS (70 eV) : *m/z* (%): 244 (45) $[M]^+$, 200 (75) $[C_9H_{12}OS_2]^+$, 167 (100) $[C_9H_{11}OS]^+$, 91 (65) $[C_7H_7]^+$; elemental analysis (%) calcd for $C_{11}H_{16}O_2S_2$: C 53.97, H 6.60, S 26.24; found: C 54.01, H 6.57, S 26.08.

### 1,2-bis(2-methoxyethylthio)-4-methylbenzene (2)

Yield: 2.38 g (92 %); $R_f$=0.80 ($CHCl_3$); $n_D^{20}$: 1.575; $^1$H NMR (300 MHz, $CDCl_3$, 25 °C, TMS): $\delta$=7.26 (d,$^3J$(H,H)=7.9 Hz, 1H; CHC*H*CS), 7.13 (s, 1H; CC*H*CS), 6.96 (d, $^3J$(H,H)=7.9 Hz, 1H; CC*H*CH), 3.53-3.63 (m, 4H; $CH_2C^{j,h}H_2O$), 3.38 (s, 3H; $OC^lH_3$), 3.35 (s, 3H; $OC^mH_3$), 3.04-3.13 (m, 4H; $SC^{i,g}H_2CH_2$), 2.31 ppm (s, 3H; $CH_3C$); $^{13}$C NMR (75 MHz, $CDCl_3$, 25 °C, TMS): $\delta$=137.7 ($CH_3CCH$), 137.2 ($CHC^aS$), 132.5 ($CHC^fS$), 130.9 (CH*C*HCS), 129.6 (C*C*HCS), 127.3 (C*C*HCH), 71.0 ($CH_2C^hH_2O$), 70.9 ($CH_2C^jH_2O$), 58.7 ($OC^lH_3$), 58.6 ($OC^mH_3$), 33.2 ($SC^gH_2CH_2$), 32.5 ($SC^iH_2CH_2$), 21.0 ppm ($CH_3C$); IR (KBr): $\tilde{\nu}$=2980 (C-H), 2924 (C-H), 2876 (C-H), 2823 (C-H), 2808 (C-H), 1459 (C-C), 1114 (C-O), 1095 (C-O), 1040 $cm^{-1}$ (C-O); UV/Vis ($H_2O$): $\lambda_{(max)}$ ($\varepsilon$)=298 (1927), 248 (10224), 216 nm (18856 $mol^{-1}dm^3cm^{-1}$); MS (70 eV) : *m/z* (%): 272 (43) $[M]^+$, 214 (36) $[C_{10}H_{14}OS_2]^+$, 167 (100) $[C_9H_{11}OS]^+$, 91 (65) $[C_7H_7]^+$;

elemental analysis (%) calcd for $C_{13}H_{20}O_2S_2$: C 57.32, H 7.40, S 23.54; found: C 57.11, H 7.45, S 23.64.

**1,2-bis(2-hydroxyethylthio)benzene (3) (State of the art)**

Yield: 2.19 g (94 %); $R_f$=0.11 ($CHCl_3$); m.p. 84.5-86.0 °C; $^1$H NMR (300 MHz, $CDCl_3$, 25 °C, TMS): $\delta$=7.38-7.43 (m, 2H; CHCHCS), 7.18-7.24 (m, 2H; CHC*H*CH), 3.72 (t, $^3J$(H,H)=5.7 Hz, 4H; $CH_2CH_2O$), 3.12 (t, $^3J$(H,H)=5.7 Hz, 4H; $SCH_2CH_2$), 2.58 ppm (s, 2H; O*H*); $^{13}$C NMR (75 MHz, $CDCl_3$, 25 °C, TMS): $\delta$=136.8 (CHCS), 130.9 (CHCHCS), 127.5 (CH*C*HCH), 59.9 ($CH_2CH_2O$), 37.8 ppm ($SCH_2CH_2$); IR (KBr): $\tilde{\nu}$=3329 (O-H), 3250 (O-H), 2971 (C-H), 2954 (C-H), 2923 (C-H), 2873 (C-H), 1449 (C-C), 1057 (C-OH), 1039 (C-OH), 1023 (C-OH), 1008 $cm^{-1}$ (C-OH); UV/Vis ($H_2O$): $\lambda_{(max)}$ ($\varepsilon$)=297 (1535), 246 (10108), 214 nm (16574 $mol^{-1}dm^3cm^{-1}$); MS (70 eV) : *m/z* (%): 230 (32) $[M]^+$, 186 (26) $[C_8H_{10}OS_2]^+$, 167 (35) $[C_9H_{11}OS]^+$, 153 (100) $[C_8H_9OS]^+$; elemental analysis (%) calcd for $C_{10}H_{14}O_2S_2$: C 52.14, H 6.13, S 27.84; found: C 52.10, H 6.10, S 27.75.

**1.2-bis(2-methoxyethylthio)benzene (4)**

Yield: 2.34 g (95 %); $R_f$=0.93 ($CHCl_3$); $n_D^{20}$: 1.583; $^1$H NMR (300 MHz, $CDCl_3$, 25 °C, TMS): $\delta$=7.31-7.36 (m, 2H; CHC*H*CS), 7.13-7.19 (m, 2H; CHC*H*CH), 3.59 (t, $^3J$(H,H)=6.8 Hz, 4H; $CH_2CH_2O$), 3.37 (s, 6H, $OCH_3$), 3.11 ppm (t, $^3J$(H,H)=6.8 Hz, 4H; $SCH_2CH_2$); $^{13}$C NMR (75 MHz, $CDCl_3$, 25 °C, TMS): $\delta$=136.9 (CHCS), 129.5 (CHCHCS), 126.6 (CH*C*HCH), 70.9 ($CH_2CH_2O$), 58.7 ($OCH_3$), 32.7 ppm ($SCH_2CH_2$); IR (KBr): $\tilde{\nu}$=2980 (C-H), 2925 (C-H), 2877 (C-H), 2824 (C-H), 2808 (C-H), 1446 (C-C), 1114 (C-O), 1094 (C-O), 1042 $cm^{-1}$ (C-O); UV/Vis ($H_2O$): $\lambda_{(max)}$ ($\varepsilon$)=300 (1310), 247 (9203), 214 nm (15695 $mol^{-1}dm^3cm^{-1}$); MS (70 eV) : *m/z* (%): 258 (44) $[M]^+$, 200 (54) $[C_9H_{12}OS_2]^+$, 167 (60) $[C_9H_{11}OS]^+$, 153 (100) $[C_8H_9OS]^+$; elemental analysis (%) calcd for $C_{10}H_{14}O_2S_2$: C 55.78, H 7.02, S 24.81; found: C 55.68, H 6.99, S 24.92.

**General procedure for the synthesis of the compounds (5) and (6) (State of the art):**

A suspension of disodium (*Z*)-dicyanoethene-1,2-dithiolate (45 mmol) and a spatula tip of sodium iodide in acetone (80 mL) was heated. Then 2-bromoethanol or 2-chloroethylmethylether (91 mmol) respectively was added. The suspension was heated under reflux and stirred for 20 hours. After that the precipitate was removed and the solvent was evaporated. The residue was dissolved in chloroform and washed with water. The organic layer was dried over anhydrous $MgSO_4$ and evaporated to dryness to yield a yellow solid. This was purified by recrystallisation from diethyl ether to afford (5) as light brownish and (6) as colourless crystals.

**(*Z*)-1,2-bis(2-hydroxyethylthio)-1,2-dicyanoethene (5)**

Yield: 0.94 g (43 %); $R_f$=0.03 ($CHCl_3$); m.p. 67.5-68.5 °C; $^1$H NMR (300 MHz, $CDCl_3$, 25 °C, TMS): $\delta$=3.92 (t, $^3J$(H,H)=5.7 Hz, 4H; $CH_2CH_2O$), 3.32 ppm (t, $^3J$(H,H)=5.7 Hz, 4H; $SCH_2CH_2$), 2.04 ppm (s, 2H; O*H*); $^{13}$C NMR (75 MHz, $CDCl_3$, 25 °C, TMS): $\delta$=121.8 (C=*C*), 112.2 (CN), 61.3 ($CH_2CH_2O$), 37.8 ppm ($SCH_2CH_2$); IR (KBr) : $\tilde{\nu}$=3325 (O-H), 2210 (C≡N), 2001 (C≡N), 1492 (C=C), 1061 (C-OH), 1014 (C-OH), 999 $cm^{-1}$ (C-OH); UV/Vis ($H_2O$): $\lambda_{(max)}$ ($\varepsilon$)=341 (13902), 275 (4450), 216 nm (5896 $mol^{-1}dm^3cm^{-1}$); MS (70 eV): *m/z* (%) 230 (20) $[M]^+$, 169 (13) $[C_6H_5N_2S_2]^+$, 159 (19) $[C_5H_5NOS_2]^+$, 45 (100) $[C_2H_5O]^+$; elemental analysis (%) calcd for $C_8H_{10}N_2O_2S_2$: C 41.72, H 4.38, N 12.16, S 27.84; found: C 41.81, H 4.32, N 12.21, S 27.79.

**(*Z*)-1.2-bis(2-methoxyethylthio)-1,2-dicyanoethene (6)**

Yield: 0.76 g (31 %); $R_f$=0.660($CHCl_3$); m.p. 36.3-37.2 °C; $^1$H NMR (300 MHz, $CDCl_3$, 25 °C, TMS): $\delta$=3.66 (t, $^3J$(H,H)=5.9 Hz, 4H; $CH_2CH_2O$), 3.39 (s, 6H, $OCH_3$), 3.32 ppm (t, $^3J$(H,H)=5.9 Hz, 4H; $SCH_2CH_2$); $^{13}$C NMR (75 MHz, $CDCl_3$, 25 °C, TMS): $\delta$=121.1 (C=*C*), 112.1 (CN), 70.6 ($CH_2CH_2O$), 59.0 ($OCH_3$), 34.8 ppm ($SCH_2CH_2$); IR (KBr): $\tilde{\nu}$=2212 (C≡N), 2002 (C≡N), 1504 (C=C), 1109 (C-O), 1040 $cm^{-1}$ (C-O); UV/Vis ($H_2O$): $\lambda_{(max)}$ ($\varepsilon$)=340 (13902), 276 (4255), 215 nm (5803 $mol^{-1}dm^3cm^{-1}$); MS (70 eV) : *m/z* (%): 258 (21) $[M]^+$, 199 (4) $[C_7H_7N_2OS_2]^+$, 59 (54) $[C_3H_7O]^+$, 45 (100) $[C_2H_5O]^+$; elemental analysis (%) calcd for $C_{10}H_{14}N_2O_2S_2$: C 46.49, H 5.46, N 10.84, S 24.82; found: C 46.55, H 5.49, N 10.93, S 24.85.

**Preparation of the solvent impregnated resins (SIRs) and investigation procedure**

**Immobilisation of the extractants on solid supports**

Silica gel 60 (0.04-0.063 mm) and silica gel (0.2-1 mm) from Merck as well as Amberlite XAD-2 from Supelco

were used.

According to the data sheet provided by Sigma Aldrich (Supelco) the utilized Amberlite XAD-2 has the following characteristics:

Amberlite® XAD®-2 polymeric adsorbent is a hydrophobic crosslinked polystyrene copolymer resin, supplied as 20-60 mesh size white insoluble beads.

The typical physical properties of Amberlite® XAD®-2 are Appearance: hard, spherical opaque beads

Solids: 55%
Porosity: 0.41mL pore/mL bead
Surface Area (Min.): 300m2/g
Mean Pore Diameter: 90Å
True Wet Density: 1.02g/mL
Skeletal Density: 1.08g/mL
Bulk Density: 640g/L

The silica gel used was purchased from Merck and characterized by its paricle size range. "silica gel 60" has a particle size range from 0.04-0.063 mm and "silica gel" has a particle size range from 0.2-1 mm. According to the product data sheet both products have a bulk density of 200-800 $kg/m^3$.

Before impregnating the silica gels were washed in the following sequence: 20% $HNO_3$, deionised $H_2O$,5 M NaCl solution, deionised $H_2O$, acetone, diethyl ether. The XAD 2 was washed with: toluene, acetone, ethanol, deionised $H_2O$. Afterwards the solid supports were dried under vacuum at 105 °C.

For the loading the "dry" impregnation method [18] was used.

The extractants have been diluted in chloroform (about 25 mL per gram extractant) and contacted with the supporting material. The solvent was evaporated using a rotary evaporator giving the SIRs. These have been washed with deionised water and dried under high vacuum.

**Solutions**

All aqueous solutions were diluted with deionised water (Elga, Purelab ultra). In order to prepare the $10^{-4}$ M Pd(II) model solutions, a 1000 ppm palladium absorption standard solution in 5% (w/w) HCl from Sigma-Aldrich was used. The solution of the automotive catalyst was obtained by leaching the monolith (1 kg) with heating with aqua regia (2 L) until the evolution of the gas was finished. Afterwards this was diluted with the same volume of deionised water. This leach liquor had an oxidation potential of 1.09 V vs. SHE and the approximate concentration of free chloride ions was 2 M. The eluent was prepared with thiourea (purissimum, Germed) and 30% (w/w) hydrochloric acid (suprapur, Merck).

**Procedure**

All experiments were carried out at room temperature. For batch experiments, 15 mg ± 1 mg of the dry SIR was mixed with 3 mL of the respective feed ($10^{-4}$ M Pd) or eluent (0.5 M thiourea in 0.1 M HCl) using a Heidolph Multi Reax vibrating shaker (1700 rpm). Afterwards the mixture was centrifuged and the aqueous sample separated. In order to determine the capacities the loading (and stripping) was done in several subsequent steps until palladium was not extracted (or re-extracted, respectively) any longer. For the column tests, 100 mg ± 1 mg of the dry SIR was filled into a bond elut reservoir column from Varian (20 μm frits, dimension: 5 mm x 55 mm, capacity: 1 mL). The feed, the wash and the stripping liquids were pumped upstream through the column via a Gilson Minipuls Evolution peristaltic pump with a constant flow rate and the eluate samples were collected using a Gilson Prep FC fraction collector. The determination of the metal concentration of the samples was done with a Perkim Elmer Optima 7300 DV ICP OES (with cyclonic spray chamber and Mira Mist nebuliser), which was coupled to the oneFAST sample introduction system from ESI. Correlating the Pd(II) content in the aqueous phases before $[Pd]_0$ and after the extraction $[Pd]^*$ gave the extraction yield $E_{Pd}$ as follows:

$$E_{Pd} = \frac{[\mathrm{Pd}]_0 - [\mathrm{Pd}]^*}{[\mathrm{Pd}]_0} \cdot 100\%$$

In the column tests the capillary and the column material were washed with 0.5 L of deionised water after each

elution step. While the flow rate of the column extraction experiments varied, the elution and wash was generally done at a flow rate of 1 mL/min.

One problem of the present invention was to provide compounds that are suitable as complex forming agents for the extraction of Pd(II) at different pH values from a wide range of secondary raw materials.

The problem of the present invention is solved by providing novel monomeric unsaturated vicinal dithioethers according to general formulae (2) and (4). The compounds of the present invention proved to be useful in the selective separation of Pd(II) in the solvent extraction and solid phase extractions using SIRs. In the present invention examples are disclosed for extraction procedures wherein the compounds of the invention are utilized.

It was exemplarily shown that the disclosed novel dithioethers can be used successfully in different environments.

The disclosed embodiments are only exemplary given for media with different pH ranges. With simple tests a person skilled in the art can easily find out which of the disclosed compounds of the invention can be utilized in different application and media that differ from the investigated reaction media.

## References

1: Warshawsky, A.; Cortina, J.L.; Aguilar, M.; Jerabek, K., New developments in solvent impregnated resins. An Overview. In Solvent extraction for the 21st century, Proceedings of ISEC '99; Barcelona, Spain, July 11-16, 1999; Cox, M.; Hidalgo, M.; Valiente, M., Eds.; Society of Chemical Industry: London, 2001; Vol. 2; pp 1267-1272.

2: Malik, P., Paiva, A.P., 2010. A novel solvent extraction route for the mutual separation of platinum, palladium, and rhodium in hydrochloric acid media. Solvent Extr. Ion Exch. 28, 49-72.

3: Bernardis, F., Grant, R.A., Sherrington, D.C., React Funct. Polym. 2005, 65, 205-217.

4: Demopoulos, G.P., Can. Min.Metall Bull. 1989, 82, 165-171.

5: Cleare, M.J., Grant, R.A., Charlesworth, P., Extr. Metall. '81, Pap. Symp. 1981, 34-41.

6: Tavlarides, L.L., Bae, J.H., Lee, C.K., 1987. Solvent extraction, membranes, and ion exchange in hydrometallurgical dilute metals separation. Sep. Sci. Technol. 22, 581-617.

7: Hartley, F.R., Murray, S.G., Levason, W., Soutter, H.E., McAuliffe, C.A., Inorg. Chim. Acta 1979, 35, 265-277.

8: Ananikov, V.P., Piroyan, A.O., Gaiduk, K.A., Beletskaya, I.P., Khrustalev, V.N., Russ. J. Org. Chem. 2009, 45, 1753-1764.

9: Drexler, H.-J., Starke, I., Grotjahn, M., Klein-peter, E., Holdt, H.-J., Inorg. Chim. Acta 2001, 317, 133-142, and references therein.

10: Holdt, H.-J., Pure Appl. Chem. 1993, 65, 477-482.

11: Heber, R., Journal. f. prakt. Chemie 1976, 318, 19-25

12: Abel, E.W., Bhargava, S.K., Kite, K., Orrell, K.G., Šik, V., Williams, B.L., Polyhedron 1982, 1, 289-298.

13: Muhammed, M.; Zagorodni, A. A., Solvent extraction and materials science. In Solvent extraction for the 21st century, Proceedings of ISEC '99; Barcelona, Spain, July 11-16, 1999; Cox, M.; Hidalgo, M.; Valiente, M., Eds.; Society of Chemical Industry: London, 2001; Vol. 1; pp 9-17.

14: Al-Bazi, S.J.; Chow, A., Platinum metals - solution chemistry and separation methods (ionexchange and solvent extraction). Talanta 1984, 31, 815-836.

15: Hahn, S., Holdt, H.-J. Extraction of hexachloroplatinate from hydrochloric acid solutions with phosphorylated hexanediol polymers. React. Funct. Polym. 2012, DOI: 10.1016/j.reactfunctpolym.2012.08.004.

16: Hubicki, Z.; Wolowicz, A., A comparative study of chelating and cationic ion exchange resins for the removal of palladium(II) complexes from acidic chloride media. J. Hazard. Mater. 2009, 164, 1414-1419

17: Hubicki, Z.; Wolowicz, A., Selective adsorption of palladium(II) complexes onto the chelating ion exchange resin dowex M 4195 - kinetic studies. Solvent Extr. Ion Exch. 2010, 28, 124-159.

18: Warshawsky, A.; Cortina, J. L.; Jerabek, K., Solvent impregnated resin application on metal separation processes. In Solvent extraction and liquid membranes; Aguilar, M.; Cortina, J. L., Eds.; Taylor & Francis Group: Boca Raton, 2008; pp 301-334.

19: Sole, K.C., in Solvent extraction and liquid membranes (Eds: M. Aguilar, J.L. Cortina) Taylor & Francis Group, Boca Raton, 2008, pp.175-176.

20: Woollam, S. F., Grant, R. A., Proceedings of ISEC 2008, 2008, 281-286.

21: Holdt, H.-J., Müller, H., Kelling, A., Drexler, H.-J., Müller, T., Schwarze, T., Schilde, U., Starke, I., Z. Anorg. Allg. Chem. 2006, 623, 114-122.

22: Holdt, H.-J., Teller, J., Z. Chem, 1988, 7, 249-250.

23: Grote, M., Kettrup A., Die Entwicklung edelmetallselektiver Extraktionsmittel und ihre Anwendbarkeit in der analytischen Chemie. Fresenius Z. Anal. Chem. 1989, 335, 698-711.

24: Tavlarides, L.L.; Lee, J.S.; Gomez-Salazar, S., New materials in solvent extraction. In Solvent extraction and liquid membranes; Aguilar, M.; Cortina, J.L., Eds.; Taylor & Francis Group: Boca Raton, 2008; pp 225-260.
25: Traczyk, F.P.; Bruening, R.L.; Dale, J.B., The application of molecular recognition technology (MRT) for removal and recovery of metal ions from aqueous solutions. Schriftenr. D. GDMB 1998, 82, 269-277.
26: Bähr, D.G., Schleitzer, G., Chem. Ber. 1957, 90, 438-443
27: Lange, S.J., Sibert, J.W., Stern, C.L., Barrett A.G.M., Hoffman, B.M., Tetrahedron 1995, 51, 8175-8188.
28: Giolando, M., Kirschbaum, K., An efficient one-pot synthesis of 1,2-benzenedithiol from benzenethiol. Synthesis 1992, 5, 451-452.
29: Pederson, C.J. Cyclic polyethers and their complexes with metal salts. JACS 1967, 89, 7017-7036.
30: Traeger, J., König, J., Stedtke, A., Holdt, H.-J. Advancement of a solvent extraction system with 1.2-bis(2-methoxyethylthio)benzene for the selective separation of palladium(II) from secondary raw materials. Hydrometallurgy 2012, accepted DOI: 10.1016/j.hydromet.2012.07.002.

## Claims

1. Unsaturated vicinal dithioethers of formulae (2) and (4) namely
1,2-bis(2-methoxyethylthio)-4-methylbenzene (2)

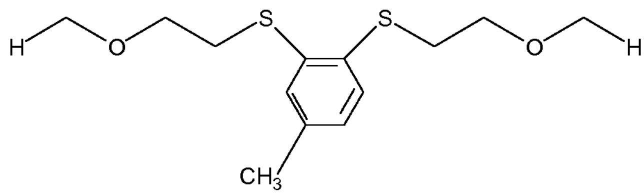

,

and
1,2-bis(2-methoxyethylthio)benzene (4)

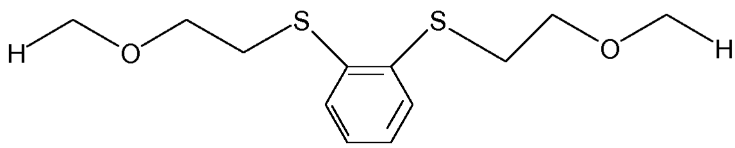

.

2. Use of a compound according to claim 1 in the separation of Pd(II) from secondary raw materials.

3. Use according to claim 2 wherein the separation is a solvent extraction method.

4. Use according to claim 2 wherein the separation is a solid phase extraction method.

5. Use according to claim 4 wherein the separation is a solvent impregnated resin (SIR) extraction method.

6. Use according to any of claims 2 to 5 wherein the secondary raw material is derived from the production or recovery of automobile catalysts.

7. Solid support comprising a compound according to claim 1.

8. Solid support according to claim 7, wherein the support is silica gel or a polymeric resin.

9. Solid support according to claim 8, wherein the polymeric resin is a macroporous styrene-divinylbenzene copolymer.

## Patentansprüche

1. Ungesättigte vicinale Dithioether der Formeln (2) und (4), nämlich 1,2-Bis(2-methoxyethylthio)-4-methylbenzol (2)

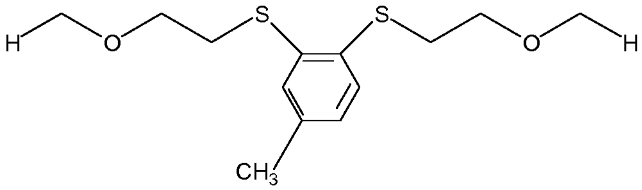

,

und

1,2-Bis(2-methoxyethylthio)benzol (4)

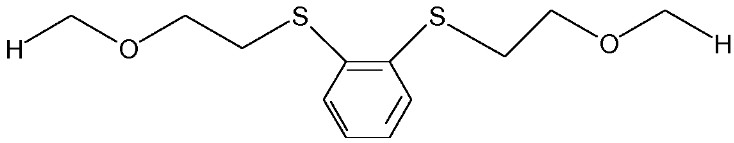

.

2. Verwendung einer Verbindung, gemäß Anspruch 1, bei der Abtrennung von Pd(II) aus sekundären Rohstoffen.

3. Verwendung, gemäß Anspruch 2, wobei die Abtrennung ein Lösungsmittelextraktionsverfahren ist.

4. Verwendung, gemäß Anspruch 2, wobei die Abtrennung ein Festphasenextraktionsverfahren ist.

5. Verwendung, gemäß Anspruch 4, wobei die Abtrennung ein Extraktionsverfahren mit einem lösungsmittelimprägnierten Harz (SIR) ist.

6. Verwendung, gemäß einem der Ansprüche 2 bis 5, wobei die sekundären Rohstoffe aus der Herstellung oder Rückgewinnung von Automobilkatalysatoren stammen.

7. Fester Träger umfassend eine Verbindung gemäß Anspruch 1.

8. Fester Träger, gemäß Anspruch 7, wobei der Träger ein Kieselgel oder ein Polymerharz ist.

9. Fester Träger, gemäß Anspruch 8, wobei das Polymerharz ein makroporöses Styrol-Divinylbenzol-Copolymer ist.

**Revendications**

1. Dithioéthers vicinaux insaturés des formules (2) et (4), à savoir le 1,2-bis(2-méthoxyéthylthio)-4-méthylbenzène (2)

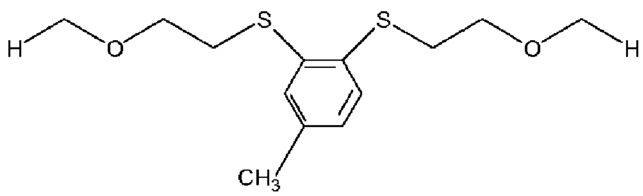

et le 1,2-bis(2-méthoxyéthylthio)benzène (4)

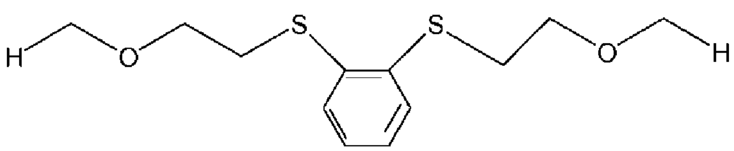

.

2. Utilisation d'un composé selon la revendication 1 dans la séparation de Pd(II) à partir de matières brutes secondaires.

**3.** Utilisation selon la revendication 2, la séparation étant un procédé d'extraction par un solvant.

**4.** Utilisation selon la revendication 2, la séparation étant un procédé d'extraction en phase solide.

**5.** Utilisation selon la revendication 4, la séparation étant un procédé d'extraction sur une résine imprégnée par un solvant (RIS).

**6.** Utilisation selon l'une quelconque des revendications 2 à 5, la matière brute secondaire provenant de la production ou de la récupération de catalyseurs automobiles.

**7.** Support solide comprenant un composé selon la revendication 1.

**8.** Support solide selon la revendication 7, le support étant un gel de silice ou une résine polymère.

**9.** Support solide selon la revendication 8, la résine polymère étant un copolymère macroporeux de styrène-divinylbenzène.

Figure 1

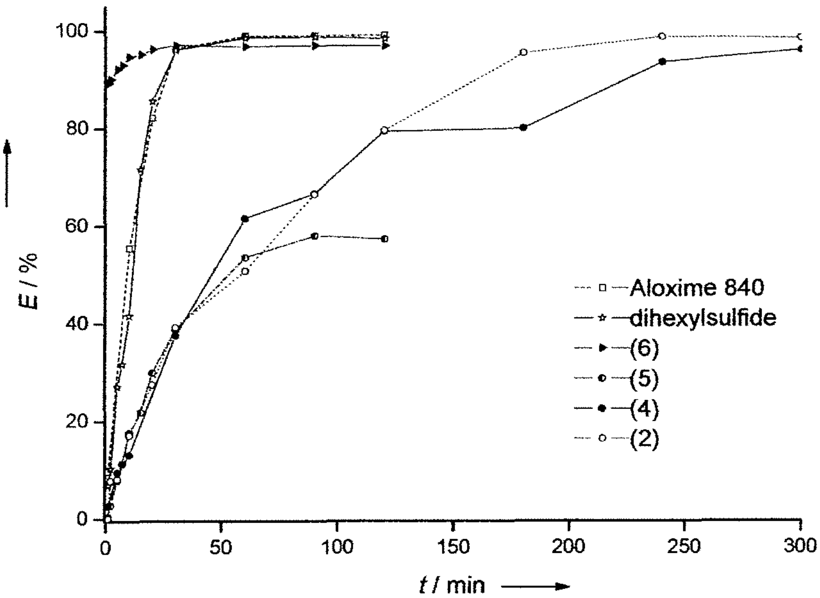


Figure 2

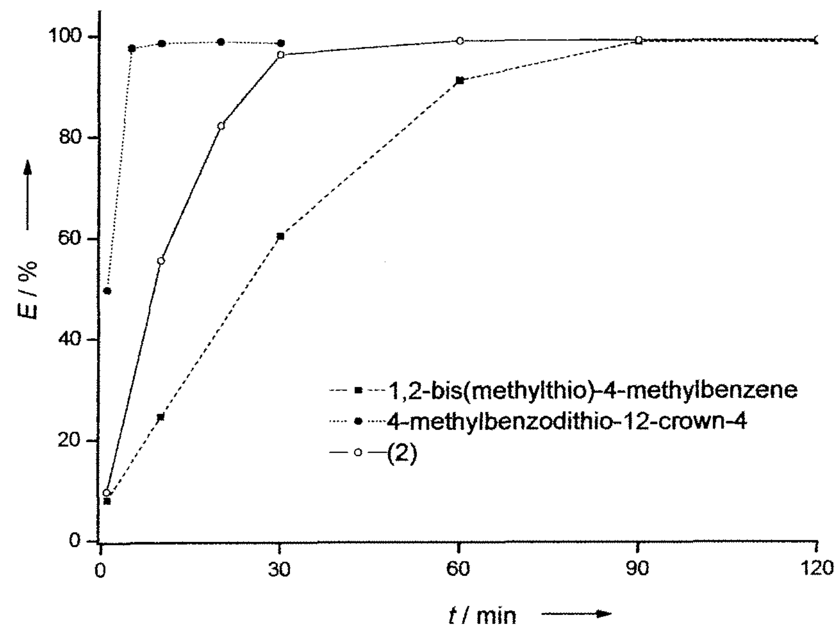

Figure 3

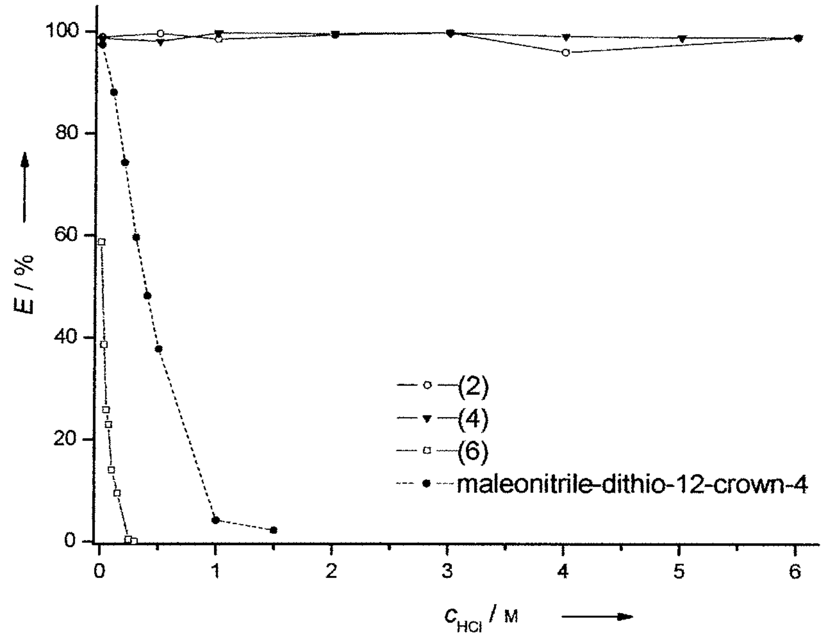


Figure 4

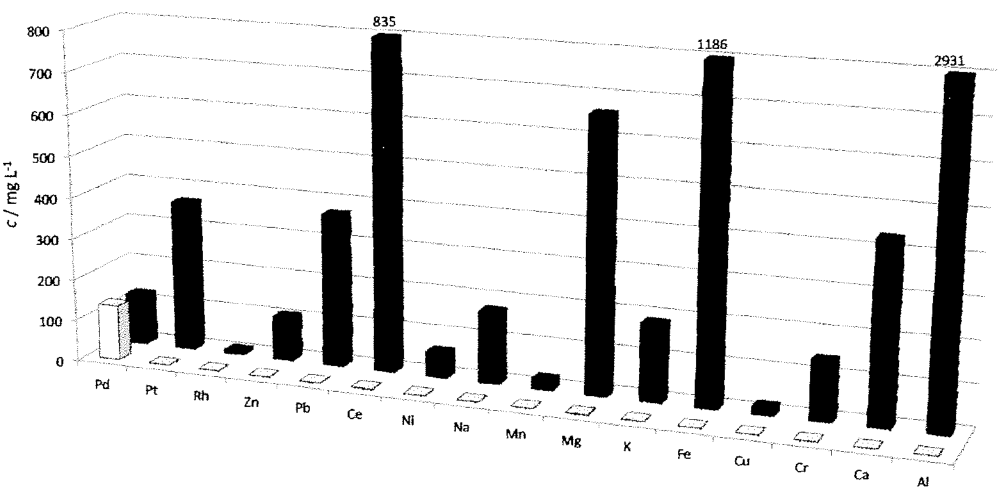

Figure 5

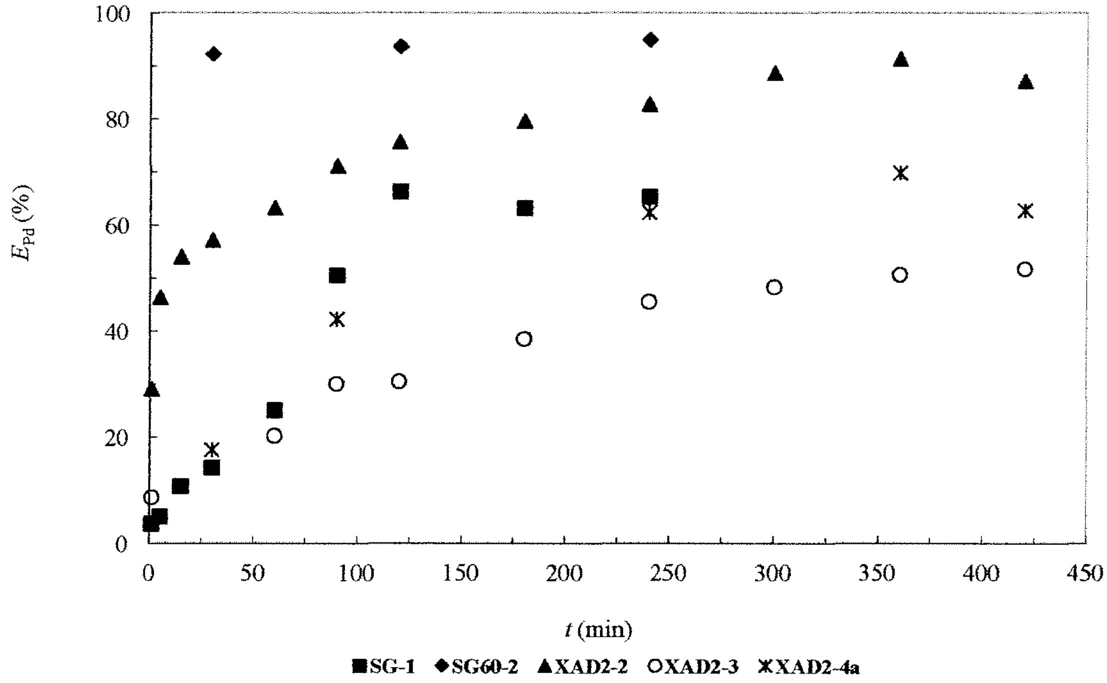


Figure 6

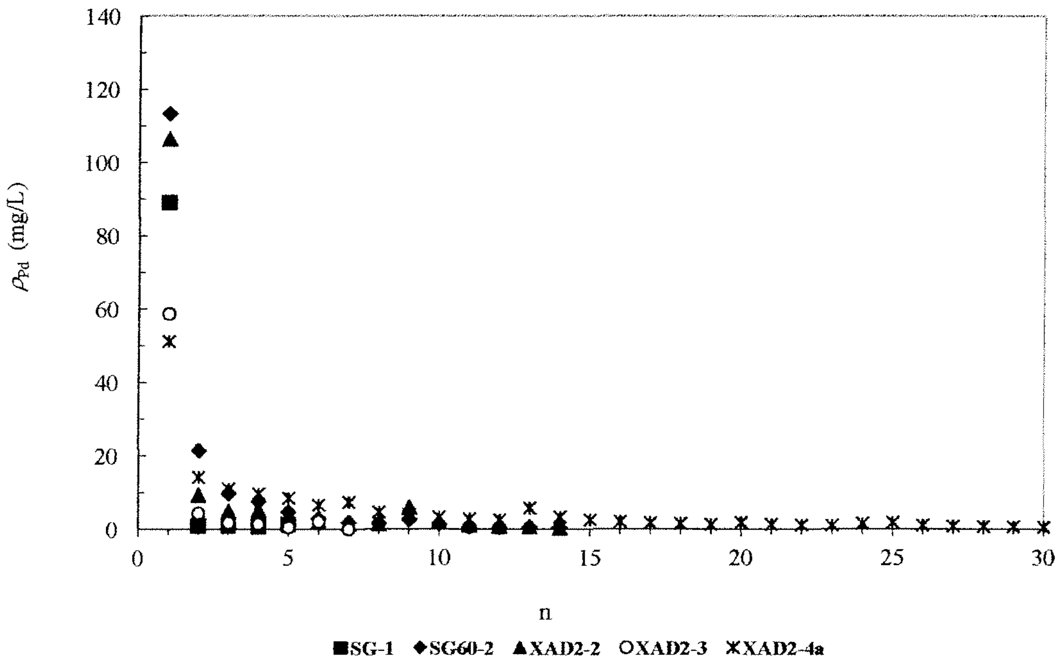

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **WARSHAWSKY, A. ; CORTINA, J.L. ; AGUILAR, M. ; JERABEK, K.** New developments in solvent impregnated resins. An Overview. *Solvent extraction for the 21st century, Proceedings of ISEC '99,* 11 July 1999
- Society of Chemical Industry. 2001, vol. 2, 1267-1272
- **MALIK, P. ; PAIVA, A.P.** A novel solvent extraction route for the mutual separation of platinum, palladium, and rhodium in hydrochloric acid media. *Solvent Extr. Ion Exch.,* 2010, vol. 28, 49-72
- **BERNARDIS, F. ; GRANT, R.A. ; SHERRINGTON, D.C.** *React Funct. Polym.,* 2005, vol. 65, 205-217
- **DEMOPOULOS, G.P.** *Can. Min.Metall Bull.,* 1989, vol. 82, 165-171
- **CLEARE, M.J. ; GRANT, R.A. ; CHARLESWORTH, P.** *Extr. Metall. '81, Pap. Symp.,* 1981, 34-41
- **TAVLARIDES, L.L. ; BAE, J.H. ; LEE, C.K.** Solvent extraction, membranes, and ion exchange in hydrometallurgical dilute metals separation. *Sep. Sci. Technol.,* 1987, vol. 22, 581-617
- **HARTLEY, F.R. ; MURRAY, S.G. ; LEVASON, W. ; SOUTTER, H.E. ; MCAULIFFE, C.A.** *Inorg. Chim. Acta,* 1979, vol. 35, 265-277
- **ANANIKOV, V.P. ; PIROYAN, A.O. ; GAIDUK, K.A. ; BELETSKAYA, I.P. ; KHRUSTALEV, V.N.** *Russ. J. Org. Chem.,* 2009, vol. 45, 1753-1764
- **DREXLER, H.-J. ; STARKE, I. ; GROTJAHN, M. ; KLEIN-PETER, E. ; HOLDT, H.-J.** *Inorg. Chim. Acta,* 2001, vol. 317, 133-142
- **HOLDT, H.-J.** *Pure Appl. Chem.,* 1993, vol. 65, 477-482
- **HEBER, R.** *Journal. f. prakt. Chemie,* 1976, vol. 318, 19-25
- **ABEL, E.W. ; BHARGAVA, S.K. ; KITE, K. ; ORRELL, K.G. ; ŠIK, V. ; WILLIAMS, B.L.** *Polyhedron,* 1982, vol. 1, 289-298
- **MUHAMMED, M. ; ZAGORODNI, A. A.** Solvent extraction and materials science. *Solvent extraction for the 21st century, Proceedings of ISEC '99,* 11 July 1999
- Society of Chemical Industry. 2001, vol. 1, 9-17
- **AL-BAZI, S.J. ; CHOW, A.** Platinum metals - solution chemistry and separation methods (ionexchange and solvent extraction). *Talanta,* 1984, vol. 31, 815-836
- **HAHN, S. ; HOLDT, H.-J.** Extraction of hexachloroplatinate from hydrochloric acid solutions with phosphorylated hexanediol polymers. *React. Funct. Polym.,* 2012
- **HUBICKI, Z. ; WOLOWICZ, A.** A comparative study of chelating and cationic ion exchange resins for the removal of palladium(II) complexes from acidic chloride media. *J. Hazard. Mater.,* 2009, vol. 164, 1414-1419
- **HUBICKI, Z. ; WOLOWICZ, A.** Selective adsorption of palladium(II) complexes onto the chelating ion exchange resin dowex M 4195 - kinetic studies. *Solvent Extr. Ion Exch.,* 2010, vol. 28, 124-159
- Solvent impregnated resin application on metal separation processes. **WARSHAWSKY, A. ; CORTINA, J. L. ; JERABEK, K.** Solvent extraction and liquid membranes. Taylor & Francis Group, 2008, 301-334
- **SOLE, K.C.** Solvent extraction and liquid membranes. Taylor & Francis Group, 2008, 175-176
- **WOOLLAM, S. F. ; GRANT, R. A.** *Proceedings of ISEC 2008,* 2008, 281-286
- **HOLDT, H.-J. ; MÜLLER, H. ; KELLING, A. ; DREXLER, H.-J. ; MÜLLER, T. ; SCHWARZE, T. ; SCHILDE, U. ; STARKE, I.** *Z. Anorg. Allg. Chem.,* 2006, vol. 623, 114-122
- **HOLDT, H.-J. ; TELLER, J.** *Z. Chem,* 1988, vol. 7, 249-250
- **GROTE, M. ; KETTRUP A.** Die Entwicklung edelmetallselektiver Extraktionsmittel und ihre Anwendbarkeit in der analytischen Chemie. *Fresenius Z. Anal. Chem.,* 1989, vol. 335, 698-711
- New materials in solvent extraction. **TAVLARIDES, L.L. ; LEE, J.S. ; GOMEZ-SALAZAR, S.** Solvent extraction and liquid membranes. Taylor & Francis Group, 2008, 225-260
- **TRACZYK, F.P. ; BRUENING, R.L. ; DALE, J.B.** The application of molecular recognition technology (MRT) for removal and recovery of metal ions from aqueous solutions. *Schriftenr. D. GDMB,* 1998, vol. 82, 269-277
- **BÄHR, D.G. ; SCHLEITZER, G.** *Chem. Ber.,* 1957, vol. 90, 438-443
- **LANGE, S.J. ; SIBERT, J.W. ; STERN, C.L. ; BARRETT A.G.M. ; HOFFMAN, B.M.** *Tetrahedron,* 1995, vol. 51, 8175-8188

- **GIOLANDO, M. ; KIRSCHBAUM, K.** An efficient one-pot synthesis of 1,2-benzenedithiol from benzenethiol. *Synthesis,* 1992, vol. 5, 451-452
- **PEDERSON, C.J.** Cyclic polyethers and their complexes with metal salts. *JACS,* 1967, vol. 89, 7017-7036
- **TRAEGER, J. ; KÖNIG, J. ; STEDTKE, A. ; HOLDT, H.-J.** Advancement of a solvent extraction system with 1.2-bis(2-methoxyethylthio)benzene for the selective separation of palladium(II) from secondary raw materials. *Hydrometallurgy,* 2012